# EUROPEAN PATENT APPLICATION

(11) **EP 2 781 198 A1**
(43) Date of publication of application: **24.09.2014**
(21) Application number: 12849910.0
(22) Date of filing: 16.11.2012
(51) Int. Cl.: A61B 18/00

(54) **ULTRASONIC VIBRATION PROBE, METHOD FOR MANUFACTURING ULTRASONIC VIBRATION PROBE AND ULTRASONIC THERAPEUTIC DEVICE**

(30) Priority: 17.11.2011 JP 2011251530
(71) Applicant: Olympus Corporation, Shibuya-ku Tokyo 151-0072 (JP)
(72) Inventor: TANIGUCHI Hirofumi, Tokyo 151-0072 (JP)
(74) Representative: Kuhnen & Wacker
(86) International application number: PCT/JP2012/079809
(87) International publication number: WO 2013/073675

(57) **Abstract**

This ultrasonic oscillation probe is an ultrasonic oscillation probe for transmitting ultrasonic vibration. A first region where the crystal grain size is relatively small and a second region where the crystal grain size is relatively large are respectively formed in at least one place. A method for manufacturing an ultrasonic oscillation probe respectively forms a first region where the crystal grain size is relatively small and a second region where the crystal grain size is relatively large in at least one place and heats a portion of the ultrasonic oscillation probe to a grain coarsening temperature or higher of a material that forms the ultrasonic oscillation probe, to form the second region.

## Description

### Technical Field

The present invention relates to an ultrasonic oscillation probe that transmits ultrasonic vibrations, a method for manufacturing the ultrasonic oscillation probe, and an ultrasonic treatment apparatus including the ultrasonic oscillation probe.

Priority is claimed on Japanese Patent Application No. 2011-251530, filed November 17, 2011, the content of which is incorporated herein by reference.

### Background Art

In recent years, ultrasonic treatment apparatuss that perform a surgical operation on an affected part of a living body by using ultrasonic vibration have been widely used. For example, Patent Document 1 discloses an ultrasonic treatment apparatus that amplifies ultrasonic vibration generated from an ultrasonic oscillator with a horn of an ultrasonic oscillation probe, transmits the amplified ultrasonic vibration to a distal end of the ultrasonic oscillation probe, and performs treatment by the ultrasonic vibration. By using such a device, it is possible to press the distal end of the ultrasonic oscillation probe against the affected part of the living body to perform excision or the like of the affected part by the ultrasonic vibration.

The ultrasonic oscillation probe is a part that amplifies ultrasonic vibration generated in an ultrasonic vibrator with the horn of the ultrasonic oscillation probe, and transmits the ultrasonic vibration to the distal end (affected part of the living body) of the ultrasonic oscillation probe. Regarding the performance required of the ultrasonic oscillation probe, durability against the ultrasonic vibration, the characteristic of stably and efficiently transmitting the vibration, excellent compatibility with the living body, and the like are required. As materials that satisfy these required characteristics, pure titanium and titanium-based alloys are used. Since the pure titanium and titanium-based alloys have higher strength, the durability and the compatibility with the living body are also excellent.

### Citation List

### Patent Literature

[Patent Document 1] Japanese Patent No. 2532780

### Summary of the Invention

### Technical Problem

Incidentally, the ultrasonic oscillation probe is self-heated by the transmitted ultrasonic vibration. The self-heating is particularly remarkable in the horn that amplifies the ultrasonic vibration. If the temperature of the ultrasonic oscillation probe becomes high due to the self-heating, problems occur such that the ultrasonic oscillation probe may deteriorate, a resonant frequency may deviate, and the vibration may stop, or the ultrasonic oscillation probe itself may break.

The present invention has been made in view of the aforementioned circumstances, and an object thereof is to provide an ultrasonic oscillation probe that suppresses heat generated due to ultrasonic vibration and has excellent durability, a method for manufacturing the ultrasonic oscillation probe, and an ultrasonic treatment apparatus including the ultrasonic oscillation probe.

### Solution to Problem

As a result of close study in order to solve the above problems, the present inventors have found that heat generated by ultrasonic vibration can be suppressed by coarsening crystal grain size in a metal structure, and have completed the present invention.

According to a first aspect of the present invention, in an ultrasonic oscillation probe for transmitting ultrasonic vibration, a first region where a crystal grain size is relatively small and a second region where the crystal grain size is relatively large are respectively formed in at least one place.

According to a second aspect of the present invention, the ultrasonic oscillation probe of the first aspect may include a proximal end connected to an ultrasonic oscillator that generates the ultrasonic vibration; and a distal end that exerts the ultrasonic vibration transmitted from the proximal end to the outside, and one or more sets of the first region and the second region may be alternately arranged along a direction toward the distal end from the proximal end.

According to a third aspect and a fourth aspect of the present invention, the ultrasonic oscillation probe of the first aspect or the second aspect may include a horn that amplifies the ultrasonic vibration; and a probe that transmits the amplified ultrasonic vibration, and the crystal grain size of the horn and the crystal grain size of the probe may be different from each other.

According to a fifth aspect of the present invention, in the ultrasonic oscillation probe of the third aspect, the crystal grain size of the horn may be larger than the crystal grain size of the probe.

According to a sixth aspect of the present invention, the ultrasonic oscillation probe of any one to the first aspect to the fifth aspect may contain pure titanium.

According to a seventh aspect of the present invention, the ultrasonic oscillation probe of any one to the first aspect to the fifth aspect may contain a titanium alloy.

According to an eighth aspect of the present invention, in the ultrasonic oscillation probe of any one to the third aspect to the fifth aspect, the horn may contain an aluminum alloy, and the probe may contain a titanium alloy.

According to a ninth aspect of the present invention, in the ultrasonic oscillation probe of any one to the first aspect to the fifth aspect, the second region may be formed by being heated to a grain coarsening temperature or higher of a material that forms the ultrasonic oscillation probe.

According to a tenth aspect of the present invention, in the ultrasonic oscillation probe of any one to the first aspect to the fifth aspect, the second region may be formed by being heated to and forged at a grain coarsening temperature or higher of a material that forms the ultrasonic oscillation probe.

According to an eleventh aspect of the present invention, in a method for manufacturing an ultrasonic oscillation probe for transmitting ultrasonic vibration, the method includes the steps of: respectively forming a first region where a crystal grain size is relatively small and a second region where the crystal grain size is relatively large in at least one place, and heating a portion of the ultrasonic oscillation probe to a grain coarsening temperature or higher of a material that forms the ultrasonic oscillation probe, to form the second region.

Even in the method for manufacturing the ultrasonic oscillation probes of the second aspect to the fifth aspect, the second region may be formed by heating a portion of the ultrasonic oscillation probe to a grain coarsening temperature or higher of a material that forms the ultrasonic oscillation probe.

According to a twelfth aspect of the present invention, in the method for manufacturing an ultrasonic oscillation probe of the eleventh aspect, in heating, the second region may be formed by heating and forging a portion of the ultrasonic oscillation probe.

An ultrasonic treatment apparatus according to a thirteenth aspect includes an ultrasonic oscillator that generates the ultrasonic vibration; and the ultrasonic oscillation probe according to any one of the first aspect to the fourth aspect that is connected to the ultrasonic oscillator.

### Advantageous Effects of Invention

According to the above ultrasonic oscillation probe, a region where the crystal grain size is relatively small and a region where the crystal grain size is relatively large are respectively formed in at least one place. Thus, heat generated during the ultrasonic vibration in the region where the crystal grain size is large can be suppressed, and the durability can be improved.

Additionally, according to the above method for manufacturing the ultrasonic oscillation probe, the ultrasonic oscillation probe is configured so that the ultrasonic oscillation probe is partially heated to a temperature or higher at which the crystal grain size is coarsened. Thus, the region where the crystal grain size is relatively large can be reliably formed.

### Brief Description of Drawings

FIG. 1A is an overall view of an ultrasonic treatment apparatus related to an embodiment of the present invention.
FIG. 1B is an enlarged view of a distal end of an ultrasonic oscillation probe.
FIG. 2 is a schematic explanatory view of the ultrasonic oscillation probe related to the embodiment of the present invention.
FIG. 3A is an optical microscope photograph of a metal structure of a horn in the ultrasonic oscillation probe.
FIG. 3B is an optical microscope photograph of the metal structure of the horn in the ultrasonic oscillation probe.
FIG. 4A is an optical microscope photograph of the metal structure of a distal end of the probe in the ultrasonic oscillation probe.
FIG. 4B is an optical microscope photograph of the metal structure of the distal end of the probe in the ultrasonic oscillation probe.
FIG. 5A is a schematic explanatory view of a method for manufacturing the ultrasonic oscillation probe related to the embodiment of the present invention.
FIG. 5B is a schematic explanatory view of the method for manufacturing the ultrasonic oscillation probe.
FIG. 5C is a schematic explanatory view of the method for manufacturing the ultrasonic oscillation probe.
FIG. 6 is a schematic explanatory view of the ultrasonic oscillation probe related to the embodiment of the present invention in which one or more sets of a region where the crystal grain size is relatively large and a region where the crystal grain size is relatively small are alternately arranged.

### Description of Embodiments

An embodiment of the present invention will be described below with reference to the drawings.

The present embodiment relates to an ultrasonic oscillation probe that amplifies ultrasonic vibration to transmit the vibrations to a probe distal end, a method for manufacturing the ultrasonic oscillation probe, and an ultrasonic treatment apparatus including the ultrasonic oscillation probe.

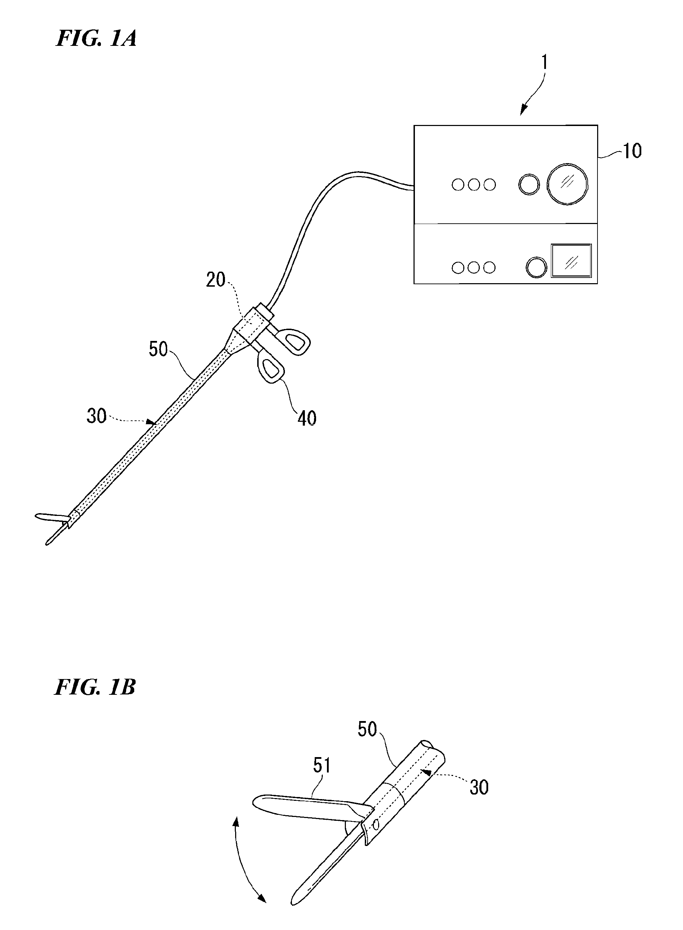

An ultrasonic treatment apparatus 1 of the present embodiment, as shown in FIG. 1A, includes a main body section 10, an ultrasonic oscillator 20 connected to the main body section 10, an ultrasonic oscillation probe 30 connected to the ultrasonic oscillator 20, and a handle 40 that grips the ultrasonic oscillation probe 30 with a hand. Also, the ultrasonic oscillator 20 and the ultrasonic oscillation probe 30 are covered with a covering portion 50.

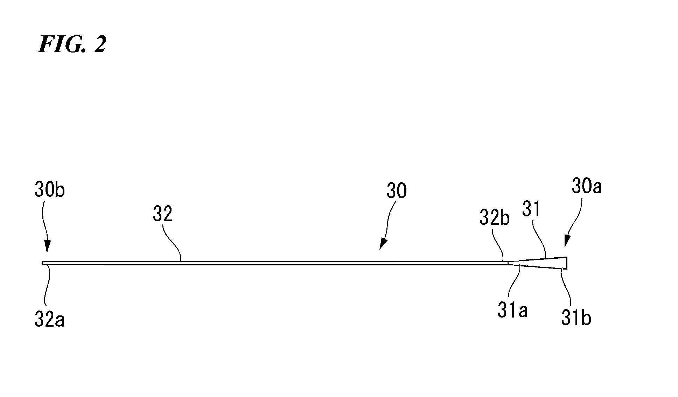

The main body section 10 has a controller that controls the operation of the ultrasonic oscillator 20 that generates ultrasonic waves. The ultrasonic oscillation probe 30 has a proximal end connected to the ultrasonic oscillator 20, and a distal end that exerts ultrasonic vibration transmitted from the proximal end operate. In addition, in the present embodiment, the ultrasonic vibration generated from the ultrasonic oscillator 20, as indicated by the arrow of FIG. 2, is a longitudinal wave that vibrates from the proximal end of the ultrasonic oscillation probe 30 toward the distal end (in a longitudinal direction of the ultrasonic oscillation probe 30 in the present embodiment) thereof.

The distal end of the covering portion 50, as shown in FIG 1B, is provided with a jaw portion 51, and the jaw portion 51 is movable in directions of both arrows of FIG. 1B by operating the handle 40.

The ultrasonic oscillation probe 30, as shown in FIG. 2, includes the horn 31 that is provided at a proximal end 30a and decreases in diameter gradually toward a first end 31a from a second end 31b (a left side in FIG. 2), and a probe 32 that is provided at a distal end 30b and extends toward the distal end 30b side from the first end 31a of the horn 31.

The horn 31 has the second end (the right side in FIG. 2) 31b connected to the above-described ultrasonic oscillator 20, and has a configuration in which the ultrasonic vibration is transmitted from the second end 31 b of the horn 31 to the first end 31 a.

The horn 31 described above is formed in a shape that gradually decreases in diameter toward the first end 31a (that is, toward a direction in which the ultrasonic vibration is transmitted) from the second end 31b. By virtue of this configuration, the ultrasonic vibration is amplified while being transmitted through the horn 31.

In the present embodiment, the horn 31 is made of higher-strength metallic materials, such as titanium alloys including a 64 titanium alloy, pure titanium, and duralumin. When titanium alloys and pure titanium are used for the horn 31, particularly, strength is high and the durability is excellent. Additionally, when aluminum alloys represented by duralumin are used for the horn 31, costs can be reduced.

A second end 32b of the probe 32 is coupled to the first end 31a of the horn 31, and has a shape that extends toward the distal end 30b side. In the present embodiment, the external diameter of the probe 32 is constant from the first end 32a to the second end 32b. The probe 32, specifically, performs treatment, inspection, or the like as the ultrasonic vibration amplified by the horn 31 is transmitted to the distal end of the probe 32 and the distal end of the probe 32 is pressed against an object. In the present embodiment, the probe 32 is made of metallic materials having higher strength and excellent biocompatibility, such as titanium alloys, such as a 64 titanium alloy, pure titanium, or the like.

Also, in the ultrasonic oscillation probe 30 of the present embodiment, a region (a first region) where the crystal grain size is relatively small and a region (a second region) where the crystal grain size is relatively large are arranged along a direction (the longitudinal direction of the ultrasonic oscillation probe 30) that goes from the proximal end 30a of the ultrasonic oscillation probe 30 to the distal end 30b thereof. In the present embodiment, the crystal grain size of the above-described horn 31 is large compared to the crystal grain size of the probe 32. That is, the horn 31 is formed with a region where crystal grains are coarsened and the crystal grain size is relatively large, compared to the probe 32.

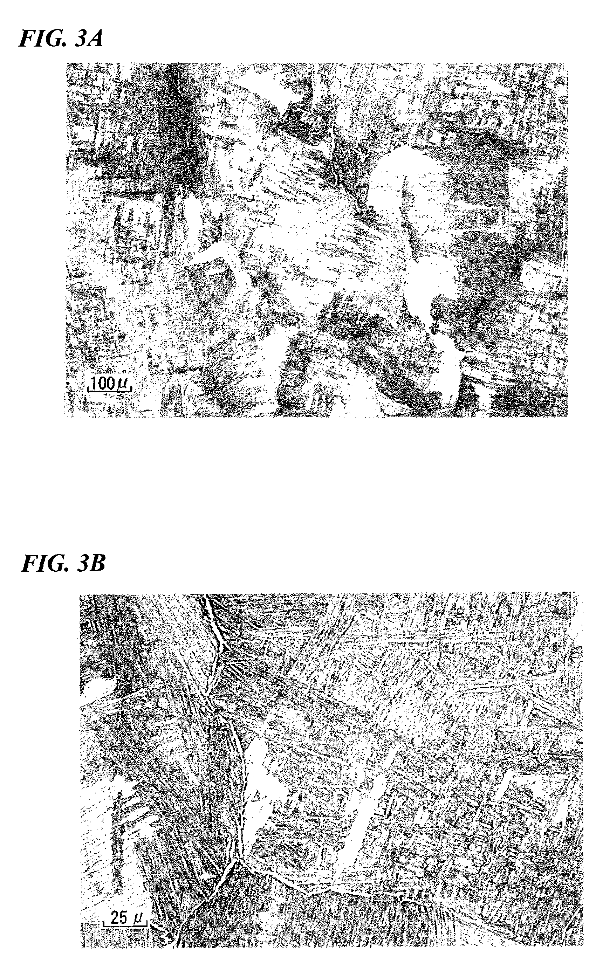

Specifically, in the present embodiment, in the case of the 64 titanium alloy, the crystal grain size of the horn 31 is set to be equal to or larger than 3 µm and equal to or smaller than 500 µm. More preferably, the crystal grain size of the horn is equal to or larger than 3 µm and equal to or smaller than 300 µm. As specific examples, optical microscope photographs of a metal structure of one end of the horn are shown in FIGS. 3A and 3B.

FIGS. 3A and 3B are optical microscope photographs obtained by observing the planes of the horn in a direction perpendicular to the longitudinal direction of the ultrasonic oscillation probe 30. In FIGS. 3A and 3B, coarse grains configured by accumulation of acicular structures are observed with a size that is equal to or larger than 100 µm.

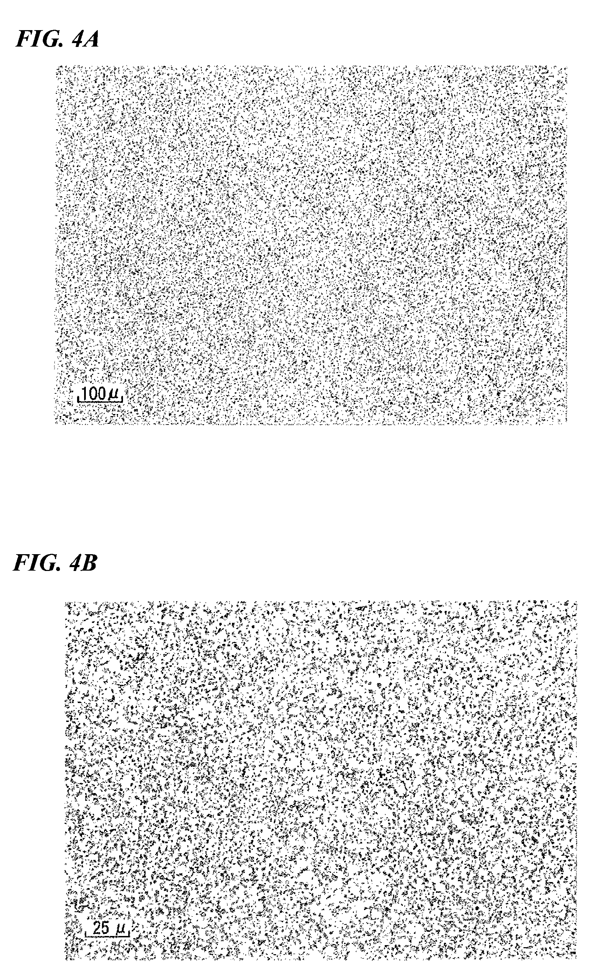

Additionally, the crystal grain size of the probe 32 is set to be equal to or smaller than 2 µm. More preferably, the crystal grain size of the probe is equal to or smaller than 1 µm. As specific examples, optical microscope photographs of the metal structure of the probe 32 are shown in FIGS. 4A and 4B. In addition, FIG 4A and FIG 4B are optical microscope photographs obtained by observing the planes of the probe 32 in the direction perpendicular to the longitudinal direction of the ultrasonic oscillation probe 30. In FIGS. 4A and 4B, an equiaxial structure that is smaller than 2 µm is observed. In addition, when the 64 titanium alloy is industrially produced, this alloy is marketed in the shape of a sheet or a rod. When the alloy is processed in the shape of a sheet or a rod, it is common that the crystal grains are made fine and have a crystal grain size of 2 µm or smaller.

Next, a method for manufacturing the ultrasonic oscillation probe 30 of the present embodiment will be described.

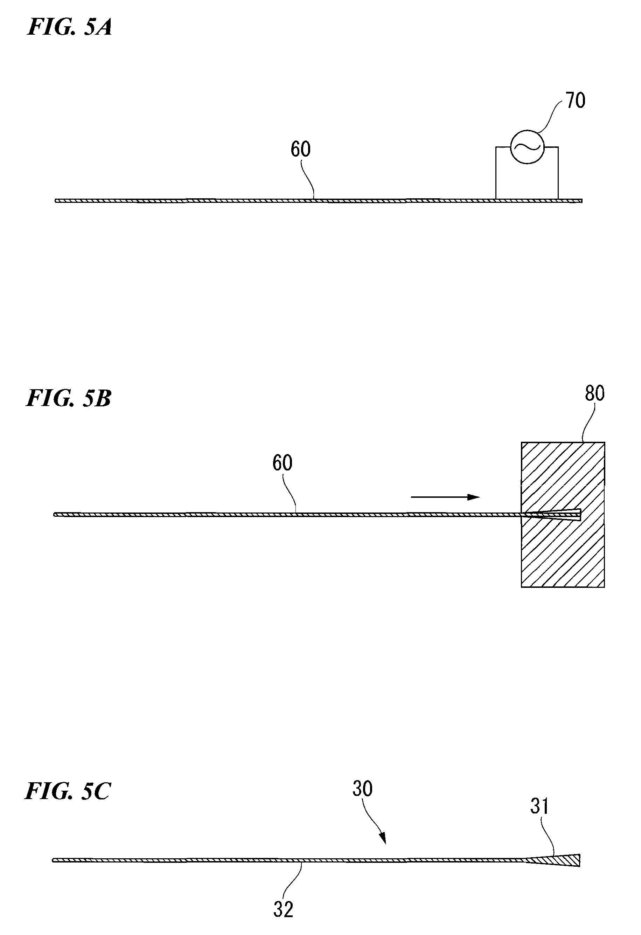

The ultrasonic oscillation probe 30 of the present embodiment is manufactured by an electric upsetting, which is a kind of forging. The electric upsetting, as shown in FIGS. 5A to 5C, is a processing method of allowing electricity to flow to a processing site of a metallic rod material 60 from the power source 70 to heat the processing region (electric heating) (FIG. 5A) and of pushing the processing region into a mold 80 to compress the processing region in a longitudinal direction of the metallic rod material 60 to expand the processing region in a radial direction (FIG. 5B) to obtain a desired shape (FIG. 5C). That is, heat influence on other sites can be suppressed by heating only the processing site.

In the present embodiment, a portion of the rod material is heated to a grain coarsening temperature (transformation temperature of a metallic material) or higher, at which crystal grains of the metallic material that constitutes the ultrasonic oscillation probe 30 are coarsened, and the upset processing is performed to make the horn 31 having a desired crystal grain size. Specifically, the temperature at which the crystal grains are coarsened is equal to or higher than 990°C in the case of 64 titanium, is equal to or higher than 885°C in the case of pure titanium, and is equal to or higher than 480°C in the case of 7075-T6 of duralumin. Then, the rod material is cut with a predetermined length to obtain the ultrasonic oscillation probe 30 having the horn 31 and the probe 32. In this way, the crystal grains of the horn 31 are made coarser than the crystal grains of the probe 32, and form the region where the crystal grain size is relatively large.

When oxidization or the like has occurred on the surface of the ultrasonic oscillation probe 30 during the processing, an oxide layers may be removed by pickling, cutting, or the like. Additionally, in order to suppress the oxidization of the surface layer, heat treatment may be performed in a vacuum atmosphere or an argon gas atmosphere.

The ultrasonic oscillation probe 30 having such a configuration is used in the ultrasonic treatment apparatus 1 shown in FIGS. 1A and 1B.

Next, a method for using the ultrasonic treatment apparatus 1 will be described. First, the main body section 10 is operated to generate ultrasonic vibration from the ultrasonic oscillator 20 and transmit vibrations to the ultrasonic oscillation probe 30. Then, the ultrasonic vibration is amplified by the horn 31 and further transmitted to the distal end (one end) of the probe 32. An operator of the ultrasonic treatment apparatus 1 grips, for example, a patient's affected part to be excised, between the distal end of the ultrasonic oscillation probe 30 and the jaw portion 51 while gripping the handle 40 with a hand. Thereafter, the affected part can be excised by generating ultrasonic vibrations. Next, the ultrasonic vibration can be stopped and the handle 40 can be operated to grip the excised piece by the distal end 30b of the ultrasonic oscillation probe 30 and the jaw portion 51 and remove the excised piece from the body.

According to the ultrasonic oscillation probe 30 of the present embodiment having the configuration described above, the crystal grain size of the horn 31 is larger than the crystal grain size of the probe 32. Thus, heat generation of the horn 31 occurring during the magnification of the ultrasonic vibration can be suppressed, degradation of the ultrasonic oscillation probe 30 can be suppressed, and the durability can be improved. Additionally, since the crystal grain size of the distal end of the probe 32 is smaller than that of the horn 31, the 0.2% proof stress becomes high. Accordingly, when the ultrasonic oscillation probe 30 is used for the ultrasonic treatment apparatus 1 in practice, plastic deformation does not occur easily and the durability is excellent.

That is, the horn 31 where ultrasonic vibration is amplified is a site where the most heat is generated (vibrational energy is easily converted into heat energy) in the ultrasonic oscillation probe 30 because the vibrational energy is amplified (densified). Due to this heat generation, the horn 31 and the probe 32 have a high temperature and deteriorate easily. It is believed that this heat generation occurs due to the friction of a crystal interface caused by vibrations, and it is preferable that crystal interface per unit cross-sectional area in a plane in a direction parallel to the diameter of the horn 31 be smaller so that the heat generation of the horn 31 is suppressed. Therefore, in the present embodiment, the crystal grain size of the horn 31 is set to be equal to or larger than 3 µm and equal to or smaller than 500 µm, and more preferably, equal to or larger than 3 µm and equal to or smaller than 300 µm.

Since the crystal grain size of the horn 31 is set to be equal to or larger than 3 µm and equal to or smaller than 500 µm, the heat generation occurring during the magnification of the ultrasonic vibration can be sufficiently suppressed, and the durability can be further improved. Additionally, when the crystal grain size of the horn 31 is larger than 500 µm, the heat generation during the ultrasonic vibration is further suppressed. However, in this case, degradation of mechanical strength (0.2% proof stress or the like) is large. For example, when being used for the ultrasonic treatment apparatus, the durability degradation caused by the strength degradation occurs such that breaking occurs during use. Therefore, the upper limit of the crystal grain size is set to the above range.

Meanwhile, since the distal end (first end) 32a of the probe 32 is brought into contact with an object, the distal end 32a of the probe 32 is stressed. In order to transmit the ultrasonic vibration transmitted to the distal end to a target member (a patient's affected part or the like), it is required that the transmission of the ultrasonic transmission is not cut off when the distal end is plastically deformed even if the distal end is stressed, and the strength (0.2% proof stress) that withstands such stress is required. In order to enhance this strength, it is preferable that the metal structure be made fine. Therefore, in the present embodiment, the crystal grain size of the probe 32 is equal to or smaller than 2 µm, and more preferably, equal to or smaller than 1 µm.

In this way, since the crystal grain size of the distal end 32a of the probe 32 is set to be equal to or smaller than 2 µm, the 0.2% proof stress is high, the plastic deformation does not occur at the distal end, and the durability of the ultrasonic oscillation probe 30 can be improved.

According to the method for manufacturing the ultrasonic oscillation probe 30 of the present embodiment, the horn 31 is formed by performing heating only on the processing site. Accordingly, the crystal grain size of the horn 31 is large, and it is possible to obtain the ultrasonic oscillation probe 30 in which the crystal grain size of the probe 32 is small.

According to the ultrasonic treatment apparatus 1 of the present embodiment, since the ultrasonic oscillation probe 30 having a configuration in which the crystal grain size of the horn 31 is large and the crystal grain size of the distal end of the probe 32 is small is included, heat generation occurring in the horn 31 due to the ultrasonic vibration can be suppressed. Additionally, since the crystal grain size of the distal end 32a of the probe 32 is small, the strength is high, and the plastic deformation does not easily occur even if the distal end 32a of the probe 32 is stressed. By virtue of these, the degradation of the ultrasonic oscillation probe 30 can be suppressed and the reliability of the ultrasonic treatment apparatus 1 that performs a surgical operation or the like can be improved.

Although the ultrasonic oscillation probe, the method for manufacturing the ultrasonic oscillation probe, and the ultrasonic treatment apparatus, which is one embodiment of the present invention, have been described above, the present invention is not limited to this, and can be appropriately changed without departing from the technical idea of the present invention.

Although a case where the crystal grain size of the horn is large and the crystal grain size of the distal end of the probe is small has been described in the above embodiment, an ultrasonic oscillation probe having a configuration in which at least one region where the crystal grain size is small and at least one region where the crystal grain size is relatively large are respectively formed may be adopted.

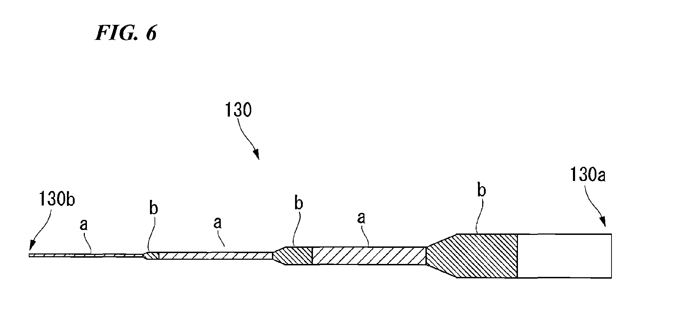

Additionally, as shown in FIG. 6, a configuration may be provided having a site in which one or more sets of a region a where the crystal grain size is small and a region b where the crystal grain size is large are alternately arranged in a direction (on a line) that goes from a proximal end 130a of the ultrasonic oscillation probe 130 to a distal end 130b. For example, as an ultrasonic treatment tool to be used for a laparoscopic operation or the like, an ultrasonic oscillation probe with different lengths according to an affected part to be treated is used. When the ultrasonic oscillation probe is long, a malfunction may occur in that the ultrasonic vibration transmitted from the ultrasonic oscillator is converted and attenuated into heat energy while being transmitted through the ultrasonic oscillation probe 130 and desired vibration is not obtained at the distal end. In order to avoid this problem, the attenuated ultrasonic vibration is amplified by providing the horn halfway from the proximal end of the ultrasonic oscillation probe 130 to the distal end thereof. Since heat generation accompanying the magnification of the ultrasonic vibration occurs at the horn halfway from the proximal end of the probe to the distal end, it is preferable that the horn include the region where the crystal grain size is relatively large, and thereby, the heat generation can be suppressed. Since portions excluding the horn may include the region where the crystal grain size is small, a configuration is provided in which the region a where the crystal grain size is small and the region b where the crystal grain size is large are alternately arranged. In addition, a plurality of the horns provided halfway from the proximal end of the probe to the distal end thereof may be provided.

Due to the reasons described above, the configuration shown in FIG. 6 in which one or more sets of the region a where the crystal grain size is small and the region b where the crystal grain size is large are alternately arranged may be required. One set means a combination of a region where the crystal grain size is relatively small and a region where the crystal grain size is relatively large.

Additionally, although a case where the probe is formed by the upset processing has been described in the above embodiment, the horn and the probe may be made by performing cutting work on a metallic material heated to a temperature or higher at which crystal grains are coarsened.

Additionally, the horn and the probe may be made by performing cutting work on a metallic material, and then, heat treatment may be performed on a predetermined site at a temperature or higher at which crystal grains are coarsened.

Additionally, the ultrasonic oscillation probe may be made by performing cutting work after a metallic material in which crystal grains are coarsened by being heated to a temperature or higher at which the crystal grains are coarsened, and a metallic material with fine structure are joined.

Additionally, although the method of performing heat treatment on a metallic material at a temperature or higher at which crystal grains are coarsened, and of manufacturing the ultrasonic oscillation probe by the electric upset method has been described, other hot forging methods may be used.

Additionally, although a case where the heat treatment method when the ultrasonic oscillation probe is manufactured is electric heating has been described in the above embodiment, an electric furnace, a burner, high-frequency induction heating, or the like may be used.

Additionally, in the above embodiment, a configuration may be adopted in which quenching is performed by quenching means, such as water cooling after heat treatment is performed and crystal grains are coarsened.

### Examples

Although examples of the present invention will be described below in detail, the present invention is not limited to these and can be appropriately changed without departing from the technical idea.

### (Example 1)

A horn and a probe were made by using a rod material made of a commercially available 64 titanium alloy, performing the electric upsetting on one end of the rod material, and facing the surface of the rod material with cutting work to remove an oxide layers. The electric upsetting was performed under a condition in which the maximum heat treatment temperature was 1105°C (measured by a radiation thermometer). Next, an ultrasonic oscillation probe was obtained by performing vacuum heating at a highest attained temperature of 650°C in order to remove the processing stress of the surface during the facing.

### (Example 2)

A horn and a probe were made by using a rod material made of a commercially available 64 titanium alloy, performing the electric upsetting on one end of the rod material, and facing the surface of the rod material with cutting work to remove an oxide layers. The electric upsetting was performed under a condition in which the highest heat treatment temperature was 1046°C (measured by the radiation thermometer).

Next, an ultrasonic oscillation probe was obtained by performing vacuum heating at a highest arrival temperature of 650°C in order to remove the processing stress of the surface during the facing.

### (Comparative Example 1)

A horn and a probe were made by performing cutting work on a rod material made of a commercially available 64 titanium alloy. Thereafter, an ultrasonic oscillation probe was obtained by performing vacuum heating at a highest arrival temperature of 650°C.

Next, a method for evaluating the performance of the examples of the present invention will be described.

### (A) Vibration Characteristics

Second ends of the horns of the ultrasonic oscillation probes obtained on respective manufacturing conditions were connected to the ultrasonic oscillator, and the temperatures of the ultrasonic oscillation probes were measured. Continuous vibration for 5 minutes was performed with an ultrasonic wave of 60 kHz as the condition of the ultrasonic vibration, and highest heat generation temperatures in that case were performed. The temperatures were measured by the radiation thermometer.

### (B) Crystal Grain Sizes

The crystal grain sizes were measured by performing observation of the metal structure of cross-sections parallel to the diameter of the horns with an optical microscope, and conforming to JIS G 0551 using observation photographs of the optical microscope.

### (C) 0.2% proof stress

Tensile test pieces from sites where the crystal grain size is the same as the horns were made. The test method was performed according to JIS Z2201 and Z2241.

**[Table 1]**

| | Material Name | | Heat Treatment Temperature during Electric upsetting | Vibration Characteristic | Metallic Structure | Strength |
|---|---|---|---|---|---|---|
| | Horn | Probe | | Highest Arrival Temperature | Crystal Grain Size | 0.2% Proof Stress |
| | | | °C | °C | µm | MPa |
| Example 1 | 64 Titanium | 64 Titanium | 1105 | 108 | >100 | 819 |
| Example 2 | 64 Titanium | 64 Titanium | 1046 | 121 | 70 | 845 |
| Comparative Example 1 | 64 Titanium | 64 Titanium | - | 190 | <2 | 896 |

The results of evaluation of Example 1, Example 2, and Comparative Example 1 are shown in Table 1.

Since the crystal grain sizes of the horns were large in Example 1 and Example 2, the heat generation during the ultrasonic vibration was small, the 0.2% proof stress was high, and the performance was excellent. In contrast, since the crystal grain size was small in Comparative Example 1, the heat generation during the ultrasonic vibration was large, and the performance of the ultrasonic oscillation probe was inferior to Example 1 and Example 2.

### (Example 3)

A horn and a probe were made by using a rod material made of a commercially available JIS4 type pure titanium, performing the electric upsetting on one end of the rod material, and facing the surface of the rod material with cutting work to remove an oxide layers. The electric upsetting was performed under a condition in which the highest heat treatment temperature was 1468°C (measured by the radiation thermometer). Next, an ultrasonic oscillation probe was obtained by performing vacuum heating at a highest arrival temperature of 650°C in order to remove the processing stress of the surface during the facing.

### (Comparative Example 2)

A horn and a probe were made by performing cutting work on a commercially available JIS4 type rod material. Thereafter, an ultrasonic oscillation probe was obtained by performing vacuum heating at a highest arrival temperature of 650°C.

**[Table 2]**

| | Material Name | | Heat Treatment Temperature during Electric upsetting | Vibration Characteristic | Metallic Structure | Strength |
|---|---|---|---|---|---|---|
| | Horn | Probe | | Highest Arrival Temperature | Crystal Grain Size | 0.2% Proof Stress |
| | | | °C | °C | µm | MPa |
| Example 3 | Pure Titanium | Pure Titanium | 1468 | 130 | >100 | 479 |
| Comparative Example 2 | Pure Titanium | Pure Titanium | - | 175 | <2 | 492 |

The results obtained by performing the above evaluation test on Example 3 and Comparative Example 2 are shown in Table 2.

Since the crystal grain size is large in Example 3, the highest arrival temperature during the ultrasonic vibration was low, the 0.2% proof stress was high, and the performance was excellent. In contrast, since the crystal grain size was small in Comparative Example 2, the highest arrival temperature during the ultrasonic vibration was high, and the performance was inferior to Example 3.

### (Example 4)

A horn shape was processed by using a commercially available duralumin (7075-T6) rod material, performing the electric upsetting on one end of the rod material, and facing the surface of the rod material with cutting work to remove an oxide layers. The electric upsetting was performed under a condition that the highest heat treatment temperature was 615°C (measured by the radiation thermometer). Next, a horn was obtained by performing vacuum heating at a highest arrival temperature of 420°C in order to remove the processing stress of the surface during the facing.

Next, a probe shape was processed by performing cutting work on a rod material made of a commercially available 64 titanium alloy. Vacuum heat treatment was performed at a highest arrival temperature of 650°C in order to remove the processing stress of the surface during the cutting work.

Then, an ultrasonic oscillation probe was obtained by performing coupling between one end of the made horn and a proximal end of the probe by screw clamping.

### (Comparative Example 3)

A horn shape was processed by performing cutting work on a commercially duralumin rod material. Thereafter, a horn was obtained by performing vacuum heating at a highest arrival temperature of 420°C in order to remove the processing stress of the surface during the cutting. Next, a probe shape was processed by performing cutting work on a commercially available 64 titanium alloy, and vacuum heat treatment was performed at a highest arrival temperature of 650°C in order to remove the processing stress of the surface.

**[Table 3]**

| | Material Name | | Heat Treatment Temperature during Electric upsetting | Vibration Characteristic | Metallic Structure | Strength |
|---|---|---|---|---|---|---|
| | Horn | Probe | | Highest Arrival Temperature | Crystal Grain Size | 0.2% Proof Stress |
| | | | °C | °C | µm | MPa |
| Example 4 | Duralumin | 64 Titanium | 615 | 130 | >35 | 489 |
| Comparative Example 3 | Duralumin | 64 Titanium | - | 200 | <15 | 521 |

The results obtained by performing the above evaluation test on Example 4 and Comparative Example 3 are shown in Table 3. In addition, test pieces were made from the horns and the tensile test was performed.

Since the crystal grain size is large in Example 4, the highest arrival temperature during the ultrasonic vibration was low, the 0.2% proof stress was high, and the performance was excellent. Additionally, the cost of the ultrasonic oscillation probe having such a configuration can be reduced. In contrast, since the crystal grain size of the horn was small in Comparative Example 3, the highest arrival temperature during the ultrasonic vibration was high, and the performance was inferior to Example 4.

### Industrial Applicability

According to the above ultrasonic oscillation probe, a region where the crystal grain size is relatively small and a region where the crystal grain size is relatively large are respectively formed in at least one place. Thus, heat generated during the ultrasonic vibration in the region where the crystal grain size is large can be suppressed, and the durability can be improved.

Additionally, according to the above method for manufacturing the ultrasonic oscillation probe, the ultrasonic oscillation probe is configured to be partially heated to a temperature or higher at which the crystal grain size is coarsened. Thus, the region where the crystal grain size is relatively large can be reliably formed.

### Reference Signs List

1: ULTRASONIC TREATMENT APPARATUS
30, 130: ULTRASONIC OSCILLATION PROBE
31: HORN
32: PROBE

## Claims

1. An ultrasonic oscillation probe for transmitting ultrasonic vibration,
wherein a first region where a crystal grain size is relatively small and a second region where the crystal grain size is relatively large are respectively formed in at least one place.

2. The ultrasonic oscillation probe according to Claim 1, comprising:
a proximal end which is connected to an ultrasonic oscillator that generates the ultrasonic vibration; and
a distal end which exerts the ultrasonic vibration transmitted from the proximal end to an outside,
wherein one or more sets of the first region and the second region are alternately arranged along a direction toward the distal end from the proximal end.

3. The ultrasonic oscillation probe according to Claim 1, comprising:
a horn which amplifies the ultrasonic vibration; and
a probe which transmits the amplified ultrasonic vibration,
wherein the crystal grain size of the horn and the crystal grain size of the probe are different from each other.

4. The ultrasonic oscillation probe according to Claim 2, further comprising:
a horn which amplifies the ultrasonic vibration; and
a probe which transmits the amplified ultrasonic vibration,
wherein the crystal grain size of the horn and the crystal grain size of the probe are different from each other.

5. The ultrasonic oscillation probe according to Claim 3,
wherein the crystal grain size of the horn is larger than the crystal grain size of the probe.

6. The ultrasonic oscillation probe according to any one of Claims 1 to 5, containing pure titanium.

7. The ultrasonic oscillation probe according to any one of Claims 1 to 5, containing a titanium alloy.

8. The ultrasonic oscillation probe according to any one of Claims 3 to 5,
wherein the horn contains an aluminum alloy, and
wherein the probe contains a titanium alloy.

9. The ultrasonic oscillation probe according to any one of Claims 1 to 5,
wherein the second region is formed by being heated to a grain coarsening temperature or higher of a material that forms the ultrasonic oscillation probe.

10. The ultrasonic oscillation probe according to any one of Claims 1 to 5,
wherein the second region is formed by being heated to and forged at a grain coarsening temperature or higher of a material that forms the ultrasonic oscillation probe.

11. A method for manufacturing an ultrasonic oscillation probe for transmitting ultrasonic vibration, the method comprising the steps of:
respectively forming a first region where a crystal grain size is relatively small and a second region where the crystal grain size is relatively large in at least one place, and
heating a portion of the ultrasonic oscillation probe to a grain coarsening temperature or higher of a material that forms the ultrasonic oscillation probe, to form the second region.

12. The method for manufacturing an ultrasonic oscillation probe according to Claim 11,
in heating, the second region is formed by heating and forging a portion of the ultrasonic oscillation probe.

13. An ultrasonic treatment apparatus comprising:
an ultrasonic vibration which generates section that generates the ultrasonic vibration; and
the ultrasonic oscillation probe according to any one of Claims 1 to 4 which is connected to the ultrasonic oscillator.
